# EUROPÄISCHE PATENTSCHRIFT

(11) **EP 1 079 776 B1**
(45) Veröffentlichungstag und Bekanntmachung des Hinweises auf die Patenterteilung: **15.09.2004**
(21) Anmeldenummer: 99936279.1
(22) Anmeldetag: 19.05.1999
(51) Int. Cl.: A61F 9/007

(54) **OPERATIONSSYSTEM**
OPERATION SYSTEM
SYSTEME POUR OPERATION CHIRURGICALE

(30) Priorität: 20.05.1998 DE 19822734; 26.06.1998 DE 19828677
(43) Veröffentlichungstag der Anmeldung: 07.03.2001
(73) Patentinhaber: Koch, Hans-Reinhard, Prof. Dr., 53129 Bonn (DE)
(72) Erfinder: Koch, Hans-Reinhard, Prof. Dr., 53129 Bonn (DE)
(74) Vertreter: Czybulka, Uwe, Dipl.-Phys.
(86) Internationale Anmeldenummer: PCT/DE1999/001499
(87) Internationale Veröffentlichungsnummer: WO 1999/059510

(56) Entgegenhaltungen:
- EP-A- 0 424 686
- WO-A-98/08442
- US-A- 5 249 121

## Beschreibung

Die Erfindung bezieht sich auf ein Operationssystem, insbesondere ein ophthalmologisches Operationssystem gemäß dem Oberbegriff des Patentanspruches 1.

In den vergangenen Jahren hat in allen chirurgischen Bereichen die Bedeutung von mikro-chirurgischen Operationen erheblich zugenommen. Charakteristisch für solche Operationen ist es, dass mit mehreren unterschiedlichen Operationsinstrumenten gearbeitet wird, die oftmals rasch gewechselt werden müssen.

Als Beispiel sei die Entfernung der durch den Grauen Star getrübten natürlichen Augenlinse und deren Ersatz durch eine künstliche Intraokular-Linse genannt. Bei dieser Operation werden ophthalmologische Operationssysteme, sogenannte Phakomaschinen eingesetzt. Die Phakomaschine weist eine Versorgungseinheit mit einem Netzgerät für elektrische Energie, einen Hochfrequenz-Generator, ein Schlauch- und Anschluss-System für Operationsinstrumente, sogenannte Handstücke, Infusions-Flaschen für eine Spülflüssigkeit sowie eine oder mehrere Pumpen auf. Falls die Handstücke mit Druckluft betrieben werden, wird auch diese zur Verfügung gestellt.

Aus der US-A-5 249 121 ist eine solche Phakomaschine bekannt, an deren Versorgungseinheit mehrere Handstücke angeschlossen sind, in diesem Falle ein piezoelektrisch angetriebenes Ultraschall-Phakoemulsifikationsgerät und ein Aspiratins- und Irrigationsgerät, des weiteren eine kleine Stablampe.

Aus der EP-A2-0 596 314 ist ein ophthalmologisches Aspirationsund Irrigationssystem bekannt, mit dem bei einer Operation der intraokulare Druck wahlweise durch Zufuhr von gasförmigem oder flüssigem Medium stabil gehalten wird. Das System weist eine Druckeinheit, eine Aspirationseinheit und eine Irrigationseinheit auf, die als auswechselbare Einschübe in einem Gehäuse ausgebildet und über externe Leitungen miteinander verbunden sind.

Durch einen kleinen Schnitt in die Hornhaut des Auges werden Handstücke in das Augeninnere eingeführt, mit denen zunächst der Kapselsack, in dem sich die natürliche Augenlinse befindet, aufgetrennt, anschließend die natürliche Augenlinse durch ein Ultraschall-Phakoemulsifikationsgerät, das sogenannte Phakohandstück zertrümmert und die Bruchstücke mit einem Aspirations- und Irrigationsgerät mit Hilfe der Spülflüssigkeit abgesaugt werden. Als Handstücke werden noch bipolare Hochfrequenz-Koagulatoren eingesetzt, um Blutungen zu stillen oder die Kapselwandung durchzutrennen etc.. Der Operateur beobachtet das Operationsfeld mit einem ophthalmologischen Stereo-Mikroskop. Er steuert die Funktion der Handstücke in der Regel mit einem Fußschalter.

Wenn im Laufe des Operationsverfahrens ein anderes Handstück benötigt wird, z.B. statt eines Phakohandstückes ein Aspirations-Irrigations-Handstück, muss das gerade benutzte Handstück von den zu der Phakomaschine führenden Versorgungsschläuch und Leitungen getrennt und das neue Handstück aufgesetzt werden. Zusätzlich muss die Versorgungseinheit neu eingestellt werden, um die Versorgung des neuen Handstückes sicherzustellen. Hierzu ist eine Steuereinheit vorgesehen, z.B. eine Tastatureingabe, ein Drehschalter oder dergleichen, mit der diese Versorgungs-Parameter für das jeweilige Operationsgerät eingestellt werden können. So ist z.B. für einen Elektrokauter nur ein elektrischer Anschluss notwendig, jedoch keine Verbindung zu einer Irrigations- oder Aspirationsleitung, d.h. allgemein einer Flüssigkeitsleitung; diese Flüssigkeitsleitungen sind jedoch für das Phako-Handstück und für ein Vitrektomie-Handstück notwendig.

Nachdem derartige Operationen im Augeninneren möglichst rasch ausgeführt werden müssen, um unnötige Irritationen des Patienten und sonstige Nebenwirkungen zu vermeiden, muss der Operateur optimale Unterstützung während der Operation und insbesondere beim Wechsel von Handstücken erhalten, damit nach Anschluss eines neuen Handstückes die hierfür notwendigen neuen Parameter an der Phakomaschine rasch und zuverlässig eingestellt werden. Dies erfolgt bisher in der Regel entweder durch den Operateur selbst oder durch eine an der Operation teilnehmende und die Handhabungen sowie den Betrieb der Phakomaschine überwachende Krankenschwester. Voraussetzung für eine rasche und erfolgreiche Operation ist daher eine optimale Zusammenarbeit des Operations-Teams.

Ein Problem bei bekannten Phakomaschinen ist die Sterilität und die Sterilisation des gesamten Operationssystemes. Muss z.B. ein Handstück wegen mangelnder Sterilität ausgewechselt werden, so dauert dieses bei herkömmlichen Phakomaschinen durch das Anlegen von neuen sterilen Schläuchen, dem Befüllen der Schläuche, dem Verlegen der Schläuche durch bestimmte Führungen in der Maschine und die Herstellung der Verbindungen zwischen der Pumpe und Drucksensoren je nach Typ der Phakomaschine fünf bis zehn Minuten. Sollte es notwendig sein, die Phakomaschine während der Operation eines Patienten oder einer Patientin umzurüsten, so ist diese Zeitspanne nicht nur für den Patienten, sondern auch für das Operations-Team sehr lang. Auch die Sterilisation von Teilen der Phakomaschine ist aufwendig; soll z.B. ein Handstück sterilisiert werden, so muss das Handstück mit seinen Anschlussschläuchen und Leitungen von der Phakomaschine getrennt und anschließend im Sterilisator sterilisiert werden. Anschließend muss ein steriles Handstück mit Schläuchen und Leitungen an die Phakomaschine angeschlossen und die Schläuche mit Spülflüssigkeiz gefüllt werden. Diese Umrüstung ist kompliziert und zeitaufwendig.

Auch der Wechsel zwischen unterschiedlichen Handstücken ist bei herkömmlichen Phakomaschinen kompliziert. Hierzu müssen ebenfalls die Schlauchverbindungen des gerade verwendeten Handstückes und die elektrischen Verbindungen gelöst und dann mit dem neuen Handstück verbunden werden. Danach muss die Maschine über die Steuereinheit neu eingestellt werden, so dass die Versorgungseinheit über die für das neue Handstück benötigten Versorgungs-Parameter informiert ist. Erst danach kann mit dem neuen Handstück weiter operiert werden. Gerade bei wiederholten Wechseln zwischen unterschiedlichen Handstücken ist das bisherige Verfahren außerordentlich zeitraubend und umständlich und kann, wenn ein spezielles Handstück dringend und schnell verwendet werden soll, auch zusätzliche Risiken z. B. bei der Neueinstellung der Phakomaschine in sich bergen.

Der Erfindung liegt die Aufgabe zugrunde, ein Operationssystem, insbesondere ein ophthalmologisches Operationssystem der in Rede stehenden Art so zu modifizieren, dass dem Operateur schnell das jeweilig von ihm benötigte Handstück zur Verfügung gestellt wird und bei einem Wechsel der Handstücke auch die Neueinstellung von etwaigen Funktionsparametern des Operationssystemes zuverlässig erfolgt. Außerdem sollen dem Benutzer zuverlässige Angaben angezeigt werden, die ihn über den Funktionszustand des Operationssystemes informieren. Insbesondere soll sicher gestellt werden, dass sämtliche Einheiten stets in einem für eine Operation geeigneten Zustand sind.

Diese Aufgaben sind gemäß der Erfindung durch die Merkmale des Patentanspruches 1 gelöst.

Demgemäß weist das Operationssystem, z. B. die erwähnte Phakomaschine, eine Versorgungseinheit mit mehreren Anschlussplätzen für jeweils ein Operationsinstrument auf. Jedes Operationsinstrument ist hierbei in einer Köchereinheit aufgenommen, die an die Versorgungseinheit angedockt werden kann. Jede Köchereinheit nimmt die für das Handstück notwendigen Anschlussschläuche und einen Aufrollmechanismus für die Anschlussschläuche auf. Sie weist ferner einen das aufgenommene Handstück identifizierenden Kodieranschluss auf, der beim Andocken der Köchereinheit an die Versorgungseinheit in einen korrespondierenden Abfrageanschluss an der Versorgungseinheit eingreift. Dieser Abfrageanschluss ist mit der die Funktion des Operationssystemes steuernden Steuereinheit verbunden, die das an dem jeweiligen Anschlussplatz angeschlossene Handstück identifiziert und die Parameter des Operationssystemes für die Funktion des jeweiligen Handstückes automatisch einstellt.

Vorzugsweise ist die Versorgungseinheit aufgeteilt in eine Basiseinheit und in eine separate, mit den Anschlussplätzen für die Köchereinheiten versehene und an die Basiseinheit ankoppelbare Verteilereinheit, in der ein Schlauchsystem zur Versorgung der Handstücke mit Flüssigkeiz und ggf. Druckluft aufgenommen ist.

Die angegebene Konstruktion des Operationssystemes ist auf vielen Gebieten der Chirurgie und vorzugsweise der Mikrochirurgie brauchbar und vorteilhaft, insbesondere bei solchen Operationen, bei denen mehrere Operationsinstrumente im Wechsel benötigt werden. Im folgenden wird beispielhaft von einem ophthalmologischen Operationssystem, einer sogenannten Phakomaschine gesprochen, ohne dass dieses einschränkend gemeint ist.

Mit einem Operationssystem gemäß der Erfindung ist es möglich, rasch, sauber und vor allem steril zu arbeiten. Bei Entnahme eines Handstückes aus dem Köcher werden die damit verbundenen Anschlussschläuche und etwaige elektrische Anschlussleitungen mitgezogen; wird das Handstück in den Köcher zurückgesteckt, werden die Anschlüsse automatisch durch den integrierten Aufrollmechanismus aufgerollt, so dass die Anschlussschläuche nicht mehr wie bisher auf einem Gerätetisch herumliegen und leicht durcheinander geraten.

Durch ein derartiges Operationssystem wird die Operation für das gesamte Operations-Team wesentlich erleichtert. So braucht der Operateur beim Wechsel auf ein neues Handstück lediglich das bisher benutzte Handstück in den Köcher zu stecken, wobei der Anschlussschlauch automatisch aufgerollt wird, und anschließend ein anderes Handstück aus einem anderen Köcher zu entnehmen, um damit die Operation fortzusetzen. Die Steuereinheit wird durch Aktivierungssensoren in den Köchern automatisch beim Zurücksetzen des bisher verwendeten Handstückes und bei er Entnahme eines neuen Handstückes über das gerade aktuelle Handstück informiert und stellt die Funktionsparameter für dieses neue Handstück automatisch ein. Ein Wechsel zwischen unterschiedlichen Handstücken ist auf diese Weise ohne Komplikationen möglich; ein umständliches Auswechseln von Handstücken wie bei bekannten Operationssystemen, etwa den erwähnten Phakomaschinen, ist nicht mehr notwendig.

Die automatische Erkennung des gerade verwendeten Handstückes über die Kodieranschlüsse schaltet ferner das Risiko aus, dass zwar das Handstück gewechselt, aber vergessen wird, das Operationssystem auf das neue Handstück einzustellen.

Insgesamt kann ein Operateur mit der Wahl der vorhandenen Handstücke sehr variabel vorgehen, ohne dass hierbei das Risiko einer falschen Einstellung des Operationssystemes eingegangen wird.

So besteht z.B. bei einer Phakomaschine die Möglichkeit, zwei Phakohandstücke mit unterschiedlichen Phakonadeln in getrennten Köchern anzudocken. Hiermit kann der Operateur die Effektivität der unterschiedlichen Phakonadeln bei einem Patienten überprüfen und das jeweilig günstigste Handstück wählen. Manchmal ist es auch sinnvoll, bei einer Operation zu unterschiedlichen Zwecken zwei verschiedene Phakonadeln bzw. Handstücke einzusetzen. Bei konventionellen Phakomaschinen sind solche Möglichkeiten praktisch nicht gegeben, da die Umrüstung auf ein anderes Handstück, wie oben erläutert, nur mit großem Zeitverlust möglich ist, so dass davon üblicherweise kein Gebrauch gemacht wird.

Ein wesentlicher Vorteil der Erfindung gegenüber herkömmlichen Operationssystemen ist es, dass die einzelnen Köchereinheiten bzw. die Köcher und die Verteilereinheit z. B. zur Reinigung, Sterilisation oder Reparatur einfach von der Basiseinheit abgenommen werden können. Anschließend wird einfach ein anderer Köcher oder eine andere Verteilereinheit an die Basiseinheit angedockt. Die Arbeit mit dem Operationssystem kann dann ohne Unterbrechung fortgesetzt werden. Die durch Reinigung, Sterilisieren und Reparaturen etc. früher bedingten Stand- und Umrüstzeiten des Operationssystemes werden auf diese Weise erheblich verringert. Die schnelle Auswechselungsmöglichkeit eines Handstückes mit seinen in dem Köcher aufgenommenen Versorgungsschläuchen verringert auch das bisher vorhandene Risiko, mit nicht ausreichend sterilisierter Ausrüstung zu arbeiten.

Zum Reinigen der abgenommenen Köcher oder der Verteilereinheit ist es zweckmäßig, eine separate Spülstation vorzusehen, an die die zu reinigenden Köcher und Verteilereinheiten nach dem gleichen Prinzip angedockt werden können, d. h. dass die Spülstation Anschlussplätze für Verteilereinheiten und für Köcher aufweist. Sobald ein Köcher oder eine Verteilereinheit an die Spülstation angeschlossen wird, wird das Schlauchsystem von Köcher oder Verteilereinheit mit Spülflüssigkeit gereinigt. Die Spülstation kann als "intelligente" Station ausgebildet sein, d.h. eine Steuereinheit aufweisen, die die Schritte zum Reinigen automatisch ausführt. Es kann vorgesehen sein, diese Spülstation mit der Steuereinheit des Operationssystemes über eine Datenleitung zu verbinden, so dass der Steuereinheit nach dem Reinigen die Kenndaten der gereinigten Einheit und der Vollzug der Reinigung mitgeteilt werden. Eine solche Mitteilung an die Steuereinheit der Phakomaschine kann auch nach der Sterilisation erfolgen. Die Information an die Steuereinheit kann natürlich auch manuell eingegeben werden; ebenso ist es möglich, dass Köcher und Verteilereinheiten einen elektronischen Speicher aufweisen, in den protokollartig die mit diesen Einheiten vorgenommenen Verfahren und Vorgänge abgespeichert werden. Beim Andocken der jeweiligen Einheit an das Operationssystem werden diese an die Steuereinheit geliefert und ggf. übernommen. Auf diese Weise erkennt die Steuereinheit, ob die angedockte Einheit ordnungsgemäß für den Gebrauch vorbereitet worden ist. Sollte z. B. versehentlich eine nicht sterilisierte Einheit angeschlossen werden, dann wird von der Steuereinheit ein Alarm und ggf. eine entsprechende Anzeige ausgegeben.

Das Operationssystem weist hierzu noch eine Zähleinrichtung auf, mit der die Anzahl von Einsätzen zumindest eines Teiles des Operationssystemes bestimmt wird. Vorzugsweise ist diese Zähleinrichtung noch mit einer Zeitnahmeeinrichtung gekoppelt, die die Einsatzzeit des mit der Zähleinrichtung überwachten Teiles mißt.

Vorzugsweise werden hierbei die einzelnen Operationsinstrumente, die einzelnen Köcher und auch die Verteilereinheit mit der Zähleinrichtung und/oder der Zeitnahmeeinrichtung versehen. Natürlich kann eine derartige Ausrüstung auch für die Versorgungseinheit vorgesehen werden.

Eine solche Ausrüstung des Operationssystemes dient insbesondere dazu, die einzelnen Teile zu überwachen und nach Überschreiten einer z.B. aufgrund von Erfahrungen ermittelten vorgegebenen Anzahl von Einsätzen bzw. nach Überschreiten einer vorgegebenen Einsatzzeit z.B. ein Warnsignal abzugeben oder die Funktion des überwachten Teiles einzuschränken bzw. zu sperren, um den Benutzer z.B. auf Wartungszeiten aufmerksam zu machen.

Die Zähleinrichtung und die Zeitnahmeeinrichtung sind für die Überwachung von Köcher und Operationsinstrument bzw. Handstück vorzugsweise im Köcher gelegen, wobei die Anzahl der Köchernutzungen und des Handstückes registriert wird. Für den Fall eines ophthalmologischen Operationssystemes könnten die Zähl- und Zeitnahmeeinrichtungen so eingestellt werden, dass z.B. nach fünfzigfacher Benutzung des Operationsinstrumentes, z.B. eines Phakohandstückes, die Phakonadel zu wechseln ist und die Anschlußschläuche für das Operationsinstrument auf Dichtigkeit überprüft werden müssen. Nach hundertfacher Benutzung des Phakohandstückes in dem Köcher könnte dann z.B. ein Hinweis erscheinen, dass der Köcher mit dem Phakohandstück zum Service an den Hersteller einzuschicken ist. Sollte dies nicht erfolgen, so kann, wie oben erwähnt, die Funktion des Phakohandstückes gesperrt werden. Auf diese Weise wird der Benutzer quasi gezwungen, die vorgegebenen Serviceleistungen, wie Wechseln der Schläuche, Einmessen des Handstückes und Sicherheitsüberprüfungen durchführen zu lassen.

Mit einer Zähleinrichtung kann auch die Anzahl von Reinigungen, Sterilisationsverfahren und Serviceleistungen gezählt und der Steuereinheit mitgeteilt werden. Dies kann sowohl für den Köcher als auch für die Verteilereinheit erfolgen. Sämtliche Daten der Zähleinrichtung und/der Zeitnahmeeinrichtung können zu jeder Zeit angezeigt werden, so dass der Benutzer sich stets ein anschauliches Bild von dem Funktionszustand des gesamten Operationssystemes machen kann.

Eine Zähleinrichtung, die die Anzahl von Reinigungen, Sterilisationen und Serviceleistungen etc. überwacht, kann ein einfaches mechanisches oder elektronisches Zählwerk sein, das automatisch etwa beim Andocken eines Köchers oder einer Verteilereinheit an eine Servicestation weitergeschaltet wird. Die Anzahl der Schaltungen kann dann entweder selbst auf dem Köcher oder der Verteilereinheit oder über die Steuereinheit an einer separaten Anzeige angezeigt werden.

Über die Zähleinrichtung kann z.B. sichergestellt werden, dass das Operationsinstrument nach jedem Einsatz vorschriftsmäßig gespült oder sterilisiert wird. Dies kann über eine entsprechende Anzeige über die Steuereinheit erfolgen; es ist jedoch auch eine Sperrung der Funktion des jeweils überwachten Teiles des Operationsinstrumentes möglich, die erst wieder aufgehoben wird, wenn das überwachte Teil vorschriftsmäßig zur Benutzung vorbereitet, d.h. z.B. gereinigt, gespült oder sterilisiert worden ist.

Des weiteren werden vorzugsweise mit den Teilen des Operationssystemes, die in gewissen Abständen sterilisiert werden müssen, Sterilisationssensoren verbunden, z.B. kleine Temperatursensoren, die feststellen, ob in einem Sterilisationsvorgang die erforderliche Sterilisationstemperatur erreicht worden ist. Erst nachdem dieses erfolgt ist, wird dann z.B. die Funktion des überwachten Teiles, vorzugsweise über die Steuereinheit, wieder freigegeben.

Gemäß der Erfindung kann das Operationssystem modulartig aufgebaut werden, wobei die Breite eines Modules gerade auf diejenige eines Köchers eingestellt wird. Durch diesen modulartigen Aufbau kann das Operationssystem praktisch beliebig zusammengesetzt werden, wodurch es auch möglich ist, das Operationssystem an Weiterentwicklungen anzupassen. So kann etwa an eine vorhandene Basiseinheit ein neues Modul angeschlossen werden, das dann für den Anschluss eines neu entwickelten Handstückes mit seiner Köchereinheit geeignet ist.

Weitere Ausgestaltungen der Erfindung gehen aus den Unteransprüchen hervor.

Die Erfindung ist in einem Ausführungsbeispiel anhand der Zeichnungen näher erläutert. In dieser stellen dar:
Fig. 1 eine Frontansicht eines Operationssystemes, und zwar einer Phakomaschine gemäß der Erfindung, die aus einer Versorgungseinheit, einer Verteilereinheit und mehreren Köchern für jeweils ein Handstück zusammengesetzt ist;
Fig. 2 eine teilweise geschnittene Seitenansicht der Phakomaschine gemäß Fig. 1;
Fig. 3 eine teilweise geschnittene Aufsicht auf die Phakomaschine;
Fig. 4 eine geschnittene Teilansicht der Phakomaschine im Bereich einer Flüssigkeitspumpe;
Fig. 5 ein Ablauf-Diagramm für eine Steuereinheit der Phakomaschine gemäß der Erfindung.

In den Figuren ist eine Phakomaschine 1 dargestellt, die aus einer Basiseinheit 2, einer daran andockbaren Verteilereinheit 3 und mehreren an die Verteilereinheit anschließbaren Köcher 4 für jeweils ein Handstück 5 besteht.

Jedes Operationsinstrument 5 weist einen Anschlußschlauch 6 auf, der auf einer Rolle 7 in den Köcher 4 aufgerollt gehalten wird; die Rolle ist vorzugsweise motorgetrieben. Je nach Art des Operationsinstrumentes weist der Anschlußschlauch Flüssigkeitsleitungen 8 und 9 sowie Elektroleitungen 10 auf. In dem vorliegenden Falle ist ein Phakohandstück mit zwei Flüssigkeitsleitungen und einem elektrischen Anschluß dargestellt. Der elektrische Anschluß kann wiederum mehrere Leitungen umfassen, z.B. Leitungen für den Betriebsstrom und Datenleitungen. Diese Leitungen sind zu einem Anschlußstecker 10a geführt, der beim Andocken des Köchers 4 in einen korrespondierenden Stecker 11 eingreift. Zumindest ein Teil der Datenleitungen führt zu einem Kodieranschluß 12 im Köcher, der in einen korrespondierenden Abfrageanschluß 13 an der Basiseinheit 2 beim Andocken des Köchers 4 eingreift. Die Anschlüsse 12 und 13 können Teile der Stecker 10a bzw. 11 bilden. Die beiden Flüssigkeitsleitungen 8 und 9 führen zu einem Anschluß 14 in dem Köcher 4, der beim Andocken des Köchers 4 in einen korrespondierenden Anschluß 15 an der Verteilereinheit eingreift. Die Anschlüsse 10 bis 15 bilden jeweils einen Anschlußplatz für einen Köcher 4 bzw. ein Handstück 5.

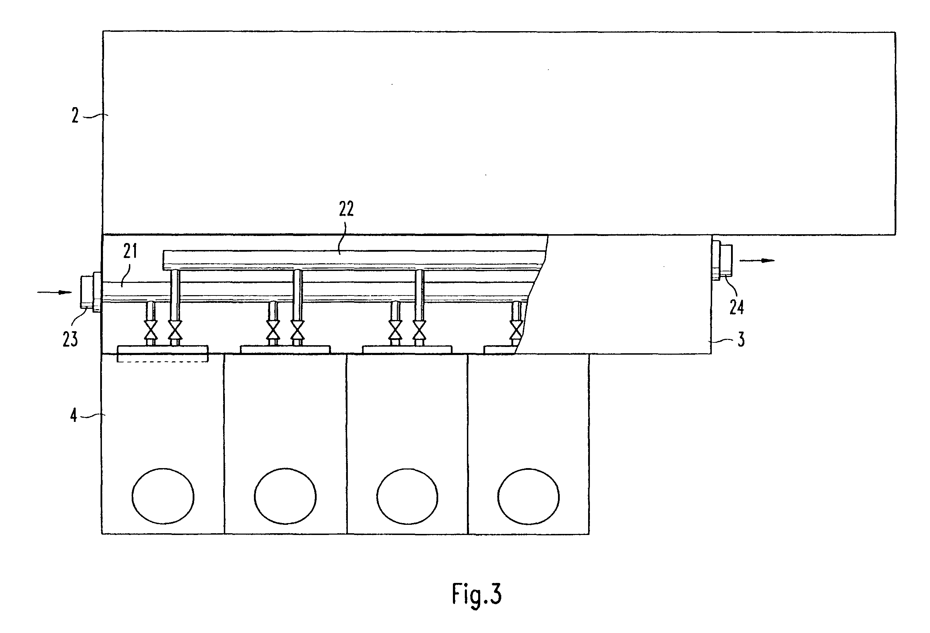

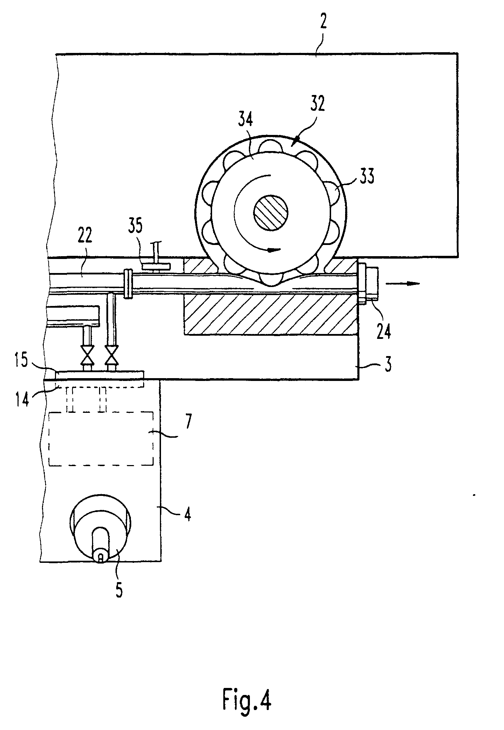

Die Verteilereinheit 3 ist, wie näher aus den Figuren 3 und 4 hervorgeht, intern mit einem Leitungs- bzw. Schlauchsystem aus einer Irrigationsleitung, d.h. einer Druckleitung 21, und einer Aspirations- bzw. Saugleitung 22 ausgerüstet, wobei die Druckleitung 21 an dem einen Ende der Verteilereinheit 3 mit einem Anschluß 23 und die Saugleitung 22 am anderen Ende der Verteilereinheit 3 ebenfalls mit einem Anschluß 24 versehen ist. Von der Druckleitung 21 zweigt an jeden Anschlußplatz eine Querleitung 25 ab, in der ein elektrisch zu betätigendes Ventil 26 liegt und die in den Anschluß 15 der Verteilereinheit 3 mündet. Von der Saugleitung 22 zweigt ebenfalls an jedem Anschlußplatz eine Querleitung 27 ab, in der ebenfalls ein elektrisch betätigbares Ventil 28 gelegen ist und die ebenfalls an jedem Anschlußplatz in den Anschluß 15 mündet. Die Leitungen sind hierbei so angeordnet, dass beim Anschließen eines Köchers 4 der Anschlußschlauch 8 mit der Querleitung 25 und der Anschlußschlauch 9 mit der Querleitung 27 verbunden wird. Mit dem Anschluß 23 am Eingang der Druckleitung 21 ist ein Schlauch 29 verbunden, der zu einer Infusionsflasche 30 führt, die ihrerseits an einem höhenverstellbaren Traggerüst 31 aufgehängt ist.

Für die Saugleitung 22 ist eine Pumpe 32 vorgesehen, die vorzugsweise als Schlauchpumpe ausgebildet ist, wie dieses schematisch in Figur 4 dargestellt ist. Die Schlauchpumpe 32 ist in der Basiseinheit 2 gelegen, greift durch eine Öffnung in der Verteilereinheit 3 in diese ein und läuft mit Wälzelementen 33 am Umfang eines Rades 34 auf dem zumindest in diesem Bereich als flexibler Schlauch ausgebildeten Saugleitung 22, so dass in dieser die Spülflüssigkeit aus der Infusionsflasche 30 abtransportiert wird. Der Anschluß 24 wird mit einem Abfluß verbunden. An die Saugleitung 22 kann noch ein Drucksensor 35 angeschlossen werden; vgl. Figur 4

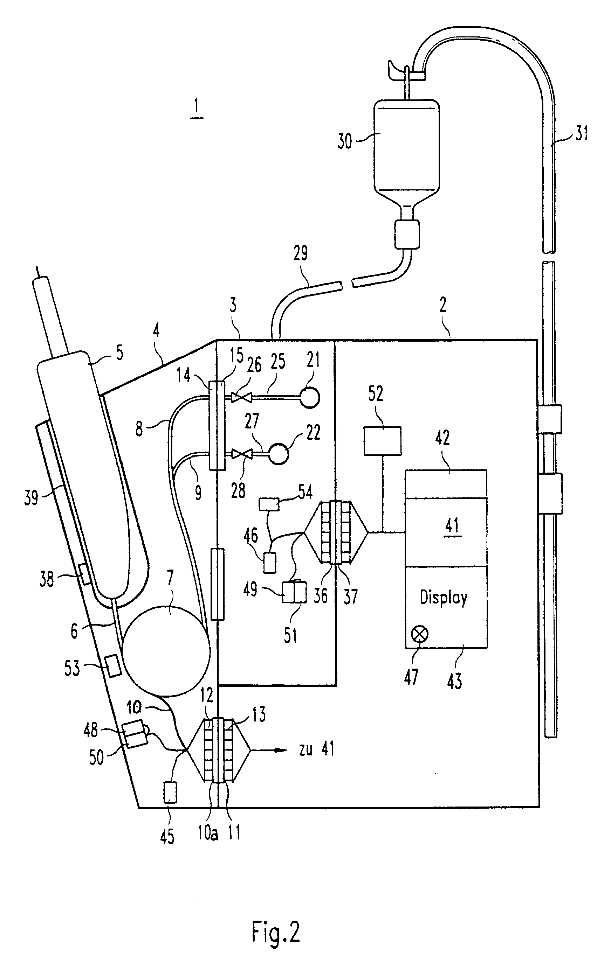

Die Verteilereinheit 3 ist, wie aus Figur 2 hervorgeht, noch mit einem elektrischen Anschluß 36 versehen, der beim Andocken der Verteilereinheit 3 an die Basiseinheit 2 in einen korrespondierenden Anschluß 37 der Basiseinheit 2 eingreift. Über diese Anschlüsse wird z.B. der Betriebsstrom für die Ventile 26 und 28 geleitet; außerdem können Datenleitungen vorgesehen sein, die etwa den jeweiligen Zustand der Ventile 26 und 28, den Füllstand in den Leitungen 21 und 22, das Ausgangssignal des Drucksensors 35 etc. melden.

In der beschriebenen Phakomaschine sind gegebenenfalls noch weitere Sensoren vorgesehen, die den Zustand der Phakomaschine erfassen; als Beispiel seien Aktivitätssensorfen 38 erwähnt, die in einer Aufnahmemulde 39 für ein Handstück 5 angeordnet sind und anzeigen, ob das Handstück 5 sich in dem Köcher 4 befindet oder aus diesem herausgenommen ist.

Ferner kann auch für das Traggerüst 31 für die Infusionsflasche 30 ein hier nur angedeuteter Sensor 40 vorgesehen sein, der die Höhe der Infusionsflasche 30 und damit auch den Druck in der Irrigationsleitung 21 angibt. Die Höhe des Traggerüstes 31 kann entweder manuell oder, wie hier nicht gezeigt, durch einen Motor eingestellt werden.

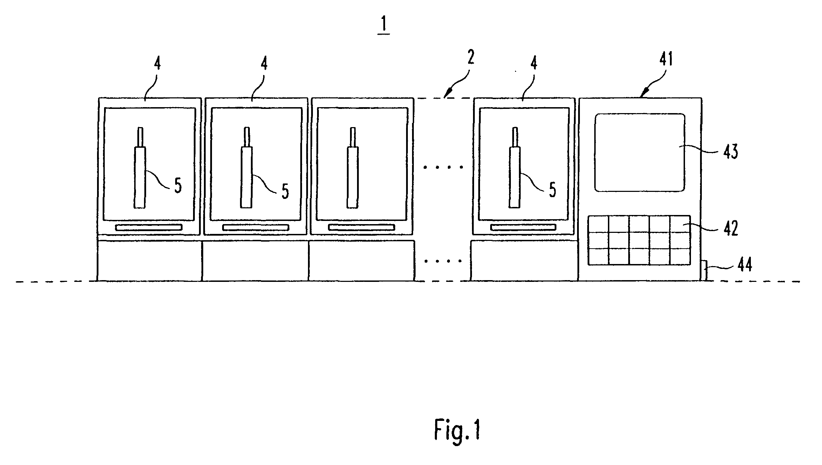

All die Signale der einzelnen Sensoren und die an den Anschlüssen 12 und 13 anliegenden Kodiersignale werden einer zentralen Steuereinheit 41 übermittelt, die Teil der Basiseinheit 2 ist, und, wie in Figur 1 angedeutet, z.B. als Modul an diese Basiseinheit angeschlossen werden kann. Aufgrund der übermittelten Signale stellt die Steuereinheit 41 die Funktionsparameter der Phakomaschine automatisch ein. Sobald dann ein Handstück 5 aus einem Köcher 4 genommen wird, wird die Versorgung für dieses Handstück entsprechend aktiviert. Hierzu gehört die Zufuhr von Spülflüssigkeit aus der Infusionsflasche 30, die Einstellung der Höhe der Infusionsflasche 30, die Einstellung der Ventile 26 und 28 für die Spülflüssigkeit, die Einstellung des Betriebsstromes für das Handstück, die Überwachung des Druckes in der Aspirationsleitung 22, die Betätigung der Schlauchpumpe 32 etc.. Die Funktion des Handstückes kann dann durch den Operateur z. B. mit dem erwähnten Fußschalter, gesteuert werden, ohne dass sonstige Einstellungen an der Phakomaschine notwendig sind. Vorteilhaft werden die Signale des Fußschalters über eine drahtlose Nachrichtenverbindung, z.B. im Funkfrequenzbereich, im Infrarot- oder im Ultraschallbereich, übertragen; auf diese Weise werden die ansonsten verwendeten, am Boden herumliegenden Kabel vermieden.

Mit der Steuereinheit 41 ist noch ein Eingabefeld 42 verbunden, z.B. in Form einer Tastatur; ferner ist eine Anzeige vorgesehen, z.B. ein Monitor 43, auf dem die Systemparameter der Phakomaschine dargestellt werden. Die Anzeige kann auch eine optische oder akustische Warnanzeige 47 umfassen. Anstelle der Tastatur 42 können natürlich auch andere Eingaben verwendet werden, so z.B. Berührungselemente direkt auf dem Monitor. Zusätzlich zum Monitor können noch über entsprechende Schnittstellen 44 Peripheriegeräte angeschlossen werden, so z.B. Drucker, mit denen ein Protokoll jeder Operation erstellt wird.

Die Köcher 4 und die Verteilereinheit 3 können noch kleine elektronische Speicher 45 bzw. 46 aufweisen, die an die Datenanschlüsse 10a bzw. 36 angeschlossen sind. In diese Speicher können Daten über die mit den Köchern und der Verteilereinheit vorgenommenen Arbeiten, z. B. Reinigungsarbeiten, Sterilisation etc. eingegeben werden, die beim Andocken eines Köchers oder der Verteilereinheit an die Steuereinheit weitergeleitet und dort ausgewertet werden, um etwa die Funktionsbereitschaft der jeweiligen Einheit zu überprüfen.

Insbesondere können im Köcher und in der Verteilereinheit jeweils eine Zähleinrichtung 48 bzw. 49 vorgesehen sein, die jeweils noch mit einer Zeitnahmeeinrichtung 50 bzw. 51 gekoppelt sind. Diese sind ebenfalls mit den Datenanschlüssen 10a bzw. 36 verbunden; eine Verbindung zu den Speichern 45 bzw. 46 kann ebenfalls vorgesehen sein. Auch in der Basiseinheit 2 kann eine Überwachungseinrichtung, vorzugsweise eine Zeitnahmeeinrichtung 52 vorgesehen sein.

Diese zusätzlichen Einrichtungen 48 bis 52 teilen ihre Daten, d.h. die Anzahl der Einsätze der einzelnen Elemente, d.h. der Handstücke, der Köcher, der Verteilereinheit und der Basiseinheit an die zentrale Steuerungseinheit 41 mit, die entsprechend vorgegebener Werte den Nutzer auf etwaig notwendige Wartungsarbeiten über die Anzeige 43 bzw. das Warnsignal 47 informiert.

In den Köcher 4 und der Verteilereinheit 3 können noch Temperatursensoren 53 und 54 vorgesehen sein, die bei einer Sterilisation dieser Teile die Temperatur erfassen und beim Andocken an die Basiseinheit 2 der Steuereinheit 41 mitteilen.

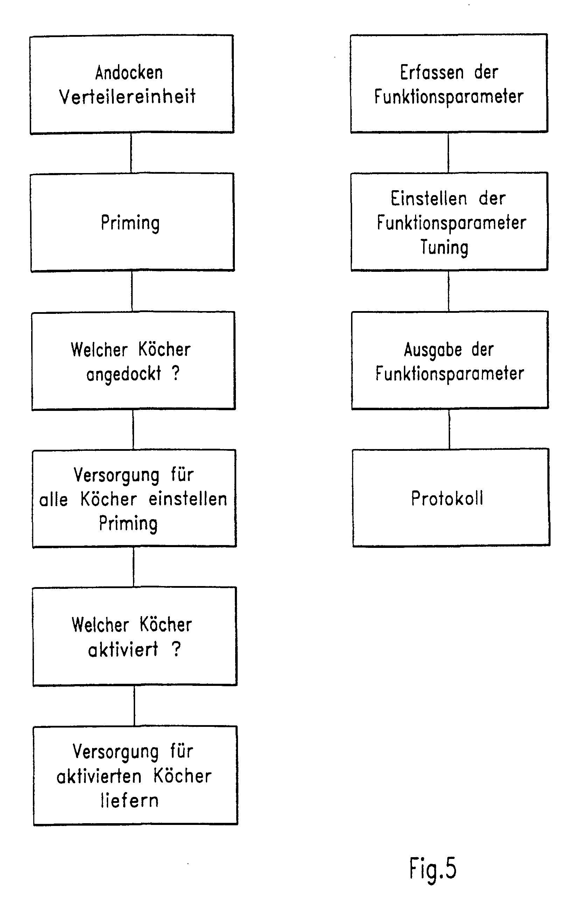

In Figur 5 ist beispielhaft ein Funktionsdiagramm für die beschriebene Phakomaschine dargestellt. Die Phakomaschine wird beim Andocken der Verteilereinheit an die Basiseinheit für eine Operation vorbereitet, indem das gesamte Schlauchsystem in der Verteilereinheit mit Spülflüssigkeit gefüllt wird. Beim Anschließen eines Köchers wird über die Kodieranschlüsse der Steuereinheit gemeldet, welche Köcher angedockt sind, d.h. es werden die jeweiligen spezifischen Daten der einzelnen Handstücke an die Steuereinheit übermittelt. Aufgrund dieser Information wird die Versorgung für alle Köcher bzw. Handstücke eingestellt, d. h. und es werden die Versorgungsschläuche in den Köchern mit Spülflüssigkeit gefüllt und ggf. an elektrische Leitungen Spannung oder an pneumatische Leitungen Druckluft angelegt. Diese vorbereitenden Vorgänge werden als "Priming" bezeichnet. Sobald dann ein Handstück aus einem Köcher genommen wird, wird die Versorgung für diesen Anschlußplatz und das entsprechende Handstück freigegeben. Die Funktion der Handstücke wird dann vom Operateur durch den hier nicht gezeigten Fußschalter gesteuert.

Parallel zu diesen Vorgängen, werden ständig die Funktionsparameter der Phakomaschine erfaßt. Diese Funktionsparameter können z.B. von dem Operateur optimiert werden, was als sogenanntes Tuning bezeichnet wird. Bei einem Phakohandstück zum Zertrümmern der natürlichen Augenlinse wird z.B. mit einer Nennfrequenz von 40 KHz gearbeitet. Um die optimale Frequenz und optimale Amplitude zu erfassen, wird das Phakohandstück zunächst in einem Testgefäß eingesetzt und optimiert; dieser Wert wird in der Steuereinheit gespeichert. Bei der Operation verändert sich jedoch die optimale Frequenz beim Berühren der natürlichen Augenlinse mit dem Phakohandstück, so dass hier ein gewisses Nachbessern oder Nachtunen erforderlich ist. Dieses Nachtunen erfolgt entweder vom Operateur oder automatisch über ein Testprogramm der Steuereinheit.

Die jeweiligen Funktionsparameter der Phakomaschine und das Protokoll zu der jeweiligen Operation werden je nach Wunsch ausgegeben oder ausgedruckt.

Wie in Figur 1 angedeutet, kann die Basiseinheit und ggf. die Verteilereinheit modulartig aufgebaut sein, wobei jedes Modul die Breite eines Köchers aufweist.

Im Vorhergehenden wurde eine Phakomaschine beschrieben, die lediglich Anschlüsse für die Spülflüssigkeit und elektrische Anschlüsse für den Betriebsstrom bzw. die Datenübertragung aufweist. Es ist natürlich möglich, das Prinzip einer solchen Phakomaschine mit Köchern auch für pneumatisch betriebene Handstücke zu verwenden. Grundsätzlich ist es möglich und vorstellbar, eine solche Phakomaschine auch mit anders gearteten Handstücken zu betreiben, z. B. mit Handstücken, mit denen die getrübte Augenlinse durch Laserstrahlung zertrümmert wird.

Außerdem sind im Vorhergehenden die Kodier- und Abfrageanschlüsse als elektrische Datenanschlüsse bezeichnet worden; es ist möglich, jede andere Art einer Kodierung zu verwenden, sofern sichergestellt ist, dass die Steuereinheit über die Art des jeweils angeschlossenen Operationsinstrumentes informiert wird. So können z.B. rein mechanische Kodierungen verwendet werden. Auch eine Kombination von elektrischen und etwa mechanischen Kodiermechanismen ist möglich.

## Patentansprüche

1. Operationssystem (1) zur Verwendung bei mikrochirurgischen Operationen, insbesondere ophtalmologisches Operationssystem, mit mehreren Operationsinstrumenten (5), die austauschbar über Anschlussschläuche (6) an eine Versorgungseinheit anschließbar sind, die die für das jeweils angeschlossene Operationsinstrument notwendige Versorgung, insbesondere mit Strom, Druckluft und/oder Flüssigkeit zur Verfügung stellt und die mehrere Anschlussplätze für jeweils ein Operationsinstrument (5) aufweist; und mit einer Steuereinheit (41), mit der Parameter für die Versorgung der Operationsinstrumente einstellbar sind, **gekennzeichnet durch** folgende Merkmale:
jedes Operationsinstrument (5) ist in einer eigenen Köchereinheit (4) aufgenommen, die an einen Anschlussplatz anschließbar ist;
jede Köchereinheit (4) weist einen, das darin aufgenommene Operationsinstrument (5) identifizierenden Kodieranschluss (12) auf, der beim Anschluss der Köchereinheit (4) an die Versorgungseinheit (2, 3) in einen Abfrageanschluss (13) der Versorgungseinheit (2, 3) eingreift;
die Steuereinheit (41) ist mit den Abfrageanschlüssen (13) an allen Anschlussplätzen verbunden, identifiziert für jeden Anschlussplatz das dort angeschlossene Operationsinstrument (5) und stellt die Parameter für dessen Versorgung ein.

2. Operationssystem nach Anspruch 1, **dadurch gekennzeichnet dass** jede Köchereinheit (4) die für das aufgenommene Operationsinstrument (5) notwendigen Anschlussschläuche (6) und einen Aufrollmechanismus (7) für diese aufweist.

3. Operationssystem nach Anspruch 1 oder 2, **dadurch gekennzeichnet, dass** die Versorgungseinheit (2, 3) aufgeteilt ist in eine Basiseinheit (2) und in eine separate, mit den Anschlussplätzen für die Köchereinheiten (4) versehene und an die Basiseinheit (2) ankoppelbare Verteilereinheit (3), die ein mit einem Zufluss (23) und einem Abfluss (24) verbundenes Schlauchsystem (21, 22) an jedem Anschlussplatz für die Versorgung der Operationsinstrumente (5) mit Spülflüssigkeit und bei druckluftbetriebenen Operationsinstrumenten mit Druckluft aufnimmt.

4. Operationssystem nach einem der vorhergehenden Ansprüche, **dadurch gekennzeichnet, dass** Aktivierungs-Sensoren (38) vorgesehen sind, die bei Entnahme des jeweiligen Operationsinstrumentes (5) aus der Köchereinheit (4) an die Steuereinheit (41) ein Aktivierungsignal abgeben, wonach die Steuereinheit (41) die Versorgung für dieses Operationsinstrument (5) aktiviert.

5. Operationssystem nach einem der vorhergehenden Ansprüche, **dadurch gekennzeichnet, dass** die Steurereineinheit (41) eine Eingabe (42) zum Programmieren der Funktion des Operationssystemes aufweist.

6. Operationssystem nach Anspruch 4, **dadurch gekennzeichnet, dass** die Steuereinheit (41) eine die Funktion des Operationssystemes darstellende Anzeige (43) aufweist.

7. Operationssystem nach einem der vorhergehenden Ansprüche, **dadurch gekennzeichnet, dass** die Steuereinheit (41) mit einer Schnittstelle (44) zum Anschluß von externen Geräten, insbesondere einem Drucker, versehen ist.

8. Operationssystem nach einem der vorhergehenden Ansprüche, **dadurch gekennzeichnet, dass** die Köchereinheit (4) und die Verteilereinheit (3) jeweils mit einem Speicher (45, 46) versehen sind, in denen spezifische Daten von Köchereinheit (4) und Verteilereinheit (5) eingebbar sind und beim Andocken an die Basiseinheit (2) an deren Steuereinheit (41) weitergeleitet werden.

9. Operationssystem nach einem der vorhergehenden Ansprüche, **dadurch gekennzeichnet, dass** das Operationssystem (1) eine Zähleinrichtung (48, 46) zum Zählen der Anzahl von Einsätzen zumindest eines Teiles (4, 5, 3) des Operationssystemes aufweist.

10. Operationssystem nach einem der vorhergehenden Ansprüche, **dadurch gekennzeichnet, dass** das Operationssystem eine Zeitnahmeeinrichtung (50, 51, 52) zum Messen der Einsatzzeit zumindest eines Teiles (4, 3, 2) des Operationssystemes (1) aufweist.

11. Operationssystem nach Anspruch 9 oder 10, **dadurch gekennzeichnet, dass** die Zähleinrichtung (48, 49) und/oder die Zeitnahmeeinrichtung (50, 51, 52) mit der zentralen Steuereinheit (41) verbunden sind.

12. Operationssystem nach einem der Ansprüche 9 bis 11, **dadurch gekennzeichnet, dass** eine Anzeige (43, 47) für die Anzahl und/oder die Zeitdauer der Einsätze des zumindest einen mit der Zähleinrichtung und/oder der Zeitnahmeeinrichtung überwachten Teiles (4, 3, 2) des Operationssystemes vorgesehen ist.

13. Operationssystem nach einem der Ansprüche 9 bis 12, **dadurch gekennzeichnet, dass** die Steuereinheit (41) nach Überschreiten einer vorgegebenen Anzahl von Einsätzen und/oder einer vorgegebenen Einsatzdauer des zumindest einen Teiles (4, 3, 2) des Operationssystemes ein Signal, insbesondere ein Warnsignal abgibt.

14. Operationssystem nach einem der Ansprüche 9 bis 13, **dadurch gekennzeichnet, dass** die Funktion des zumindest einen mit der Zähleinrichtung und/oder der Zeitnahmeeinrichtung überwachten Teiles (4, 3, 2) des Operationssystemes nach Überschreiten einer vorgegebenen Anzahl von Einsätzen und/oder vorgegebener Einsatzzeit eingeschränkt bzw. gesperrt wird.

15. Operationssystem nach einem der vorhergehenden Ansprüche, **dadurch gekennzeichnet, dass** für zu sterilisierende Teile (3, 4) des Operationssystems Temperatursensoren (53, 54) vorgesehen sind.

## Claims

1. An operating system (1) for use in microsurgical operations, in particular an ophthalmological operating system, comprising a plurality of operating instruments (5) which can be interchangeably connected by way of connecting hoses (6) to a supply unit which provides the supply necessary for the respectively connected operating instrument, in particular with current, compressed air and/or fluid, and which has a plurality of connecting locations for a respective operating instrument (5), and a control unit (41) with which it is possible to set parameters for the supply to the operating instruments, **characterised by** the following features:
each operating instrument (5) is accommodated in its own sterilising unit (4) which is connectable to a connecting location;
each sterilising unit (4) has a coding connection (12) for identifying the operating instrument (5) accommodated therein, which coding connection upon connection of the sterilising unit (4) to the supply unit (2, 3) engages into an interrogation connection (13) of the supply unit (2, 3); and
the control unit (41) is connected to the interrogation connections (13) at all connecting locations, identifies for each connecting location the operating instrument (5) connected there and sets the parameters for the supply thereof.

2. An operating system according to claim 1 **characterised in that** each sterilising unit (4) has the connecting hoses (6) necessary for the accommodated operating instrument (5) and a roll-up mechanism (7) for the hoses.

3. An operating system according to claim 1 or claim 2 **characterised in that** the supply unit (2, 3) is divided into a base unit (2) and a separate distributor unit (3) which is provided with the connecting locations for the sterilising units (4) and which can be coupled to the base unit (2) and which accommodates a hose system (21, 22) connected to a feed (23) and a discharge (24) at each connecting location for supplying the operating instruments (5) with rinsing fluid and with compressed air in the case of compressed air-operated operating instruments.

4. An operating system according to one of the preceding claims **characterised in that** there are provided activation sensors (38) which deliver an activation signal to the control unit (41) when the respective operating instrument (5) is removed from the sterilising unit (4), whereupon the control unit (41) activates the supply for said operating instrument (5).

5. An operating system according to one of the preceding claims **characterised in that** the control unit (41) has an input (42) for programming the function of the operating system.

6. An operating system according to claim 4 **characterised in that** the control unit (41) has a display (43) for displaying the function of the operating system.

7. An operating system according to one of the preceding claims **characterised in that** the control unit (41) is provided with an interface (44) for the connection of external devices, in particular a printer.

8. An operating system according to one of the preceding claims **characterised in that** the sterilising unit (4) and the distributor unit (3) are each provided with a respective memory (45, 46) in which specific data from the sterilising unit (4) and the distributor unit (5) can be inputted and can be forwarded to the control unit (41) of the base unit (2) upon docking to the base unit (2).

9. An operating system according to one of the preceding claims **characterised in that** the operating system (1) includes a counting device (48, 46) for counting the number of uses of at least a part (4, 5, 3) of the operating system.

10. An operating system according to one of the preceding claims **characterised in that** the operating system includes a timing device (50, 51, 52) for measuring the use time of at least a part (4, 3, 2) of the operating system (1).

11. An operating system according to claim 9 or claim 10 **characterised in that** the counting device (48, 49) and/or the timing device (50, 51, 52) are connected to the central control unit (41).

12. An operating system according to one of claims 9 to 11 **characterised in that** there is provided a display (43, 47) for the number and/or duration of uses of the at least one part (4, 3, 2) of the operating system, which part is monitored by the counting device and/or the timing device.

13. An operating system according to one of claims 9 to 12 **characterised in that** after a predetermined number of uses and/or a predetermined period of use of the at least one part (4, 3, 2) of the operating system is exceeded the control unit (41) outputs a signal, in particular a warning signal.

14. An operating system according to one of claims 9 to 13 **characterised in that** the function of the at least one part (4, 3, 2) of the operating system, which is monitored with the counting device and/or the timing device, is limited or blocked after a predetermined number of uses and/or a predetermined period of use is exceeded.

15. An operating system according to one of the preceding claims **characterised in that** there are provided temperature sensors (53, 54) for parts (3, 4) of the operating system, which are to be sterilised.

## Revendications

1. Système opératoire (1) à utiliser pour des opérations microchirurgicales, en particulier système opératoire ophtalmologique, comportant plusieurs instruments chirurgicaux (5) qui peuvent être raccordés de manière interchangeable et par l'intermédiaire de tuyaux de raccordement (6) à une unité d'alimentation qui fournit l'alimentation nécessaire pour l'instrument chirurgical respectivement raccordé, en particulier en courant, en air comprimé et/ou en liquide et qui présente plusieurs points de raccordement pour un instrument chirurgical (5) respectif; et comportant une unité de commande (41) par laquelle peuvent être réglés des paramètres pour l'alimentation des instruments chirurgicaux, **caractérisé par** les caractéristiques suivantes :
chaque instrument chirurgical (5) est reçu dans une propre unité de carquois (4) qui peut être raccordée à un point de raccordement ;
chaque unité de carquois (4) présente un raccord de codage (12) identifiant l'instrument chirurgical (5) qui y est reçu, lequel s'engage dans un raccord d'interrogation de l'unité d'alimentation (2, 3) lorsque l'unité de carquois (4) est raccordée à l'unité d'alimentation (2, 3) ;
l'unité de commande (41) est reliée avec les raccords d'interrogation (13) à tous les points de raccordement, elle identifie pour chaque point de raccordement l'instrument chirurgical (5) qui y est raccordé et elle règle les paramètres pour son alimentation.

2. Système opératoire selon la revendication 1, **caractérisé en ce que** chaque unité de carquois (4) présente les tuyaux de raccordement (6) nécessaires à l'instrument chirurgical (3) reçu et un mécanisme d'enroulement (7) pour ceux-ci.

3. Système opératoire selon la revendication 1 ou 2, **caractérisé en ce que** l'unité d'alimentation (2, 3) est divisée en une unité de base (2) et en une unité de distribution (9) pourvue des points de raccordement pour les unités de carquois (4) et susceptible d'être accouplée à l'unité de base (2), laquelle reçoit un système de tuyaux (21, 22) relié à une arrivée (23) et à une évacuation (24) à chaque point de raccordement pour l'alimentation des instruments chirurgicaux (5) en liquide de rinçage et en air comprimé, dans le cas d'instruments chirurgicaux fonctionnant à air comprimé.

4. Système opératoire selon l'une des revendications précédentes, **caractérisé en ce qu'**il est prévu des détecteurs d'activation (38) qui fournissent un signal d'activation à l'unité de commande (41) lorsqu'on enlève l'instrument chirurgical (5) de l'unité de carquois (4), après quoi l'unité de commande (41) active l'alimentation pour cet instrument chirurgical (5).

5. Système opératoire selon l'une des revendications précédentes, **caractérisé en ce que** l'unité de commande (41) présente une entrée (42) pour programmer la fonction du système opératoire.

6. Système opératoire selon la revendication 4, **caractérisé en ce que** l'unité de commande (41) présente un affichage (43) représentant la fonction du système opératoire.

7. Système opératoire selon l'une des revendications précédentes, **caractérisé en ce que** l'unité de commande (41) est pourvue d'une interface (44) pour le raccordement d'appareils externes, en particulier d'une imprimante.

8. Système opératoire selon l'une des revendications précédentes, **caractérisé en ce que** l'unité de carquois (4) et l'unité de distribution (3) sont chacune pourvues d'une mémoire (45, 46) dans laquelle peuvent être entrées des données spécifiques de l'unité de carquois (4) et de l'unité de distribution (3) et qui, lors de l'insertion dans l'unité de base (2), sont transmises à l'unité de commande de celle-ci.

9. Système opératoire selon l'une des revendications précédentes, **caractérisé en ce que** le système opératoire (1) présente un compteur (48, 46) pour compter le nombre d'utilisations d'au moins une partie (4, 5, 3) du système opératoire.

10. Système opératoire selon l'une des revendications précédentes, **caractérisé en ce que** le système opératoire présente un chronomètre (50, 51, 52) pour mesurer le temps d'utilisation d'au moins une partie (4, 3, 2) du système opératoire 81).

11. Système opératoire selon la revendication 9 ou 10, **caractérisé en ce que** le compteur (48, 49) et/ou le chronomètre (50, 51, 52) sont reliés à l'unité de commande (41) centrale.

12. Système opératoire selon l'une des revendications 9 à 11, **caractérisé en ce qu'**il est prévu un affichage pour le nombre et/ou pour la durée des utilisations de ladite au moins une partie (4, 3, 2) surveillée par le compteur et/ou par le chronomètre.

13. Système opératoire selon l'une des revendications 9 à 12, **caractérisé en ce qu'**après avoir dépassé un nombre prédéterminé d'utilisations et/ou après une durée d'utilisation prédéterminée de ladite au moins une partie (4, 3, 2) du système opératoire, l'unité de commande (41) fournit un signal, en particulier un signal avertisseur.

14. Système opératoire selon l'une des revendications 9 à 13, **caractérisé en ce que** la fonction de ladite au moins une partie (4, 3, 2) surveillée par le compteur et/ou par le chronomètre du système opératoire est limitée ou bloquée après avoir dépassé un nombre prédéterminé d'utilisations et/ou de temps d'utilisation prédéterminé.

15. Système opératoire selon l'une des revendications précédentes, **caractérisé en ce que** des détecteurs de température (53, 54) sont prévus pour des parties (3, 4) à stériliser du système opératoire.
